# EUROPEAN PATENT APPLICATION

(11) **EP 4 215 195 A1**
(43) Date of publication of application: **26.07.2023**
(21) Application number: 22152298.0
(22) Date of filing: 19.01.2022
(51) Int. Cl.: A61K 31/4365, A61K 31/5383, A61K 31/704, A61P 1/04, A61P 1/16, A61P 3/06, A61P 3/10, A61P 9/04, A61P 9/12, A61P 13/00, A61P 35/02, A61K 31/7048

(54) **LANATOSIDE C, CLOPIDOGREL, AND R406 IN TREATMENT OF CVD-CLONAL HEMATOPOIESIS OF INDETERMINATE POTENTIAL (CVD-CHIP)**

(71) Applicant: Genome Biologics UG, 61476 Kronberg im Taunus (DE)
(72) Inventor: KRISHNAN, Jaya, 61476 Kronberg im Taunus (DE); PHAM, Duc Minh, 63073 Offenbach (DE)
(74) Representative: Schulz Junghans Patentanwälte PartGmbB

(57) **Abstract**

The present invention relates to lanatoside C, clopidogrel, and R406 in treatment of CVD-Clonal Hematopoiesis of Indeterminate Potential (CVD-CHIP), and related diseases.

## Description

The present invention relates to lanatoside C, clopidogrel, and R406 in treatment of CVD-Clonal Hematopoiesis of Indeterminate Potential (CVD-CHIP), and related diseases.

### Background of the Invention

Clonal Hematopoiesis of Indeterminate Potential (CHIP) arises as a result of acquired mutations in a genetic allele in somatic cells, particularly hematopoietic stem cells and all their progeny cell types. It occurs when the acquired mutations lead to a proliferative or survival advantage of the cells carrying it, as well as their progeny. This causes a clonal expansion of the mutation-carrying cells, leading to them taking up a disproportionately large proportion of the cells in the blood.

CHIP has been associated with increased risk and development of cardiovascular disease (such as coronary heart disease, early onset myocardial infarction, etc.), and it is a predictor of mortality, independent of other risk factors.

The inventors have previously developed a disease model based on the most common CHIP-causing gene mutation, i.e., loss of function of TET2. The model includes a coculture of both cardiac organoids, (a state-of-the-art 3D organoid technology which replicates the physical, biochemical and physiologic properties and responses of the native human adult heart) and infiltrating myeloid cells with CHIP genotype. Together, the interaction of the two entities lead to the development of the cardiac pathology observed in CHIP in vivo, which includes increased cardiomyocyte (CM) death, inflammation, cardiac tissue fibrosis and others. The system also enables the tracking and evaluation of the aforementioned entities and their interactions. This is the first system of its kind to recapitulate the necessities for a physiologically accurate CHIP model system.

Based on the above-mentioned state of the art, the objective of the present invention is to provide means and methods for treatment for CHIP. This objective is attained by the subject-matter of the independent claims of the present specification, with further advantageous embodiments described in the dependent claims, examples, figures and general description of this specification.

### Summary of the Invention

A first aspect of the invention relates to a compound selected from lanatoside C, clopidogrel, and R406 for use in treatment of a disease associated with Cardiovascular Disease-Clonal Hematopoiesis of Indeterminate Potential (CVD-CHIP).

A further aspect of the invention relates to a compound selected from lanatoside C, clopidogrel, and R406 for use in prevention of a condition selected from myeloid cell leukemia, heart disease, atherosclerosis, hypertension, diabetes, hyperlipidemia, a high LDH level (>200 U/L), hepatic disease, renal disease, peptic ulcer disease, hypoalbuminemia (albumin level <3.5 g/dL), and thrombocytopenia (platelet count of <20 x 103 cells/µL), wherein the disease is characterized by the presence of myeloid cells comprising a mutation in TET2.

In another embodiment, the present invention relates a pharmaceutical composition comprising at least one of the compounds of the present invention or a pharmaceutically acceptable salt thereof and at least one pharmaceutically acceptable carrier, diluent or excipient.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of' or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *CHIP* in the context of the present specification relates to Clonal Hematopoiesis of Indeterminate Potential which is a condition characterized by one or several mutations in myeloid cells giving them a clonal advantage over non-mutated myeloid cells. This CHIP genotype may lead to several diseases including, but not limited to, cancer and heart disease.

The term *lanatoside* C in the context of the present specification relates to the CAS No: 17575-22-3.

The term *clopidogrel* in the context of the present specification relates to the CAS No: 135046-48-9.

The term *R406* in the context of the present specification relates to the CAS No: 841290-81-1.

### General Molecular Biology: Nucleic Acid Sequences, Expression

The term *gene* refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

The term *functional expression* in the context of the present specification refers to gene expression producing a polypeptide with capabilities of the wild-type version of the polypeptide, particularly wherein the expressed protein is the wild-type polypeptide.

The term *mutation* in the context of the present specification refers to a non-silent alteration in the nucleotide sequence of a coding region of a gene. Mutations include, but are not limited to insertions, deletions, and/or substitutions of nucleotides. Mutations include, but are not limited to loss-of-function mutations, gain-of-function mutations, and dominant-negative mutations.

The term *CHIP mutation* in the context of the present specification refers to a mutation with clonal hematopoiesis of indeterminate potential. A CHIP mutation is a mutation that happens in somatic cells (can be at any stage of cell differentiation) that leads to a clonal hematopoiesis phenotype. The phenotype is the development of a clonal population of myeloid or lymphoid cells but in the absence of detectable hematologic malignancy. The establishment of a clonal population may occur when a stem or progenitor cell acquires one or more somatic mutations that give it a competitive advantage in hematopoiesis over the stem/progenitor cells without these mutations. A variant allele frequency (VAF) of over 2% is one of the current thresholds for defining the mutation as a CHIP mutation, meaning that the mutation is found in 2% of the genetic material in the blood, and given that it happens in a single allele, this means that the mutation is observed in 4% of the cells (which have only 1 of 2 alleles mutated). Practically this means that any underlying mutation(s) which has enabled this clonal population to take over a big part of the blood cells will be a CHIP mutation. Thus, a CHIP mutation is a mutation that leads to an increased frequency of blood cells carrying this CHIP mutation inside an individual. Clonal hematopoiesis does not typically give rise to noticeable symptoms, but does lead to increased risk of cardiovascular disease. In one embodiment of the invention, the impact of CHIP mutations on the interaction of blood cells with the cardiovascular system is investigated.

The term *cardiac organoid* in the context of the present specification refers to an artificial three-dimensional structure comprising cells occurring in heart tissue, wherein the artificial three-dimensional structure mimics responses to physiological and/or biochemical and/or molecular reactions of native heart tissue.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. CHIP) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

A first aspect of the invention relates to a compound selected from lanatoside C, clopidogrel, and R406 for use in treatment of a disease associated with Cardiovascular Disease-Clonal Hematopoiesis of Indeterminate Potential (CVD-CHIP), particularly wherein the disease is characterized by the presence of myeloid cells comprising a mutation in TET2.

A further aspect of the invention relates to a compound selected from lanatoside C, clopidogrel, and R406 for use in prevention of a myeloid cell leukemia, particularly acute myeloid leukemia (AML), wherein the disease is characterized by the presence of myeloid cells comprising a mutation in TET2. The link between CVD-CHIP and myeloid cell leukemia is described in Valent et al. (Int. J. Mol. Sci. 2019, 20, 789), and in Jaiswal et al. (N Engl J Med 2014, 371;26).

A further aspect of the invention relates to a compound selected from lanatoside C, clopidogrel, and R406 for use in prevention of heart disease, wherein the disease is characterized by the presence of myeloid cells comprising a mutation in TET2. In certain embodiments, the heart disease is a condition selected from heart valve disease, cardiac inflammation, heart failure, and pathologic cardiac hypertrophy. The link between CVD-CHIP and heart disease is described in Dorsheimer et al. (JAMA Cardiol. 2019;4(1):25-33.).

A further aspect of the invention relates to a compound selected from lanatoside C, clopidogrel, and R406 for use in prevention of a condition selected from atherosclerosis, hypertension, diabetes, hyperlipidemia, and a high LDH level (>200 U/L), wherein the disease is characterized by the presence of myeloid cells comprising a mutation in TET2.

CHIP is associated with diabetes, liver disease and hyperlipidemia as described in Sorror et al. (JAMA Oncol 2017, 3, 1675) and Ferrone et al. (Int J Mol Sci 2020, 21, 626).

A further aspect of the invention relates to a compound selected from lanatoside C, clopidogrel, and R406 for use in prevention of a condition selected from hepatic disease, renal disease, peptic ulcer disease, hypoalbuminemia (albumin level <3.5 g/dL), and thrombocytopenia (platelet count of <20 x 103 cells/µL), wherein the disease is characterized by the presence of myeloid cells comprising a mutation in TET2.

In certain embodiments, the mutation in TET2 is a mutation which causes expression of a loss-of-function TET2 protein or a TET2 protein with reduced activity, or a mutation which lowers or abrogates expression of TET2. In certain embodiments, the mutation introduces a stop codon into the TET2 gene leading to expression of a loss-of-function TET2 protein.

In certain embodiments, the myeloid cells comprise functional expression of the genes FLT3, N/KRAS, KIT, PTPN11, PHF6, SRSF2, STAG2, EZH2, and RAD21.

In certain embodiments, the myeloid cells comprise functional expression of the genes PML-RARA, MYH11-CBFB, RUNX1-RUNX1T1, MLLT3-KMT2A, DEK-NUP214, RUNX1, NPM1, CEBPA, GATA2.

In certain embodiments, the compound is lanatoside C.

In certain embodiments, the compound is clopidogrel.

In certain embodiments, the compound is R406.

### Medical treatment, Dosage Forms and Salts

Similarly, within the scope of the present invention is a method or treating CHIP or an associated disease in a patient in need thereof, comprising administering to the patient a compound according to the above description.

Similarly, a dosage form for the prevention or treatment of CHIP or an associated disease is provided, comprising a compound according to any of the above aspects or embodiments of the invention.

The skilled person is aware that any specifically mentioned drug compound mentioned herein may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present.

### Pharmaceutical Compositions and Administration

Another aspect of the invention relates to a pharmaceutical composition comprising a compound (lanatoside C, clopidogrel, or R406) of the present invention, or a pharmaceutically acceptable salt thereof, and a pharmaceutically acceptable carrier. In further embodiments, the composition comprises at least two pharmaceutically acceptable carriers, such as those described herein.

In certain embodiments of the invention, the compound of the present invention is typically formulated into pharmaceutical dosage forms to provide an easily controllable dosage of the drug and to give the patient an elegant and easily handleable product.

The pharmaceutical composition can be formulated for enteral administration, particularly oral administration or rectal administration. In addition, the pharmaceutical compositions of the present invention can be made up in a solid form (including without limitation capsules, tablets, pills, granules, powders or suppositories), or in a liquid form (including without limitation solutions, suspensions or emulsions).

The pharmaceutical composition can be formulated for parenteral administration, for example by i.v. infusion, intradermal, subcutaneous or intramuscular administration.

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of Manufacture and Method of Treatment according to the invention

The invention further encompasses, as an additional aspect, the use of a compound as identified herein (lanatoside C, clopidogrel, or R406), or its pharmaceutically acceptable salt, as specified in detail above, for use in a method of manufacture of a medicament for the treatment or prevention of CHIP or an associated disease.

Similarly, the invention encompasses methods of treatment of a patient having been diagnosed with a disease associated with CHIP or an associated disease. This method entails administering to the patient an effective amount of a compound as identified herein (lanatoside C, clopidogrel, or R406), or its pharmaceutically acceptable salt, as specified in detail herein.

### CHIP mutations

**Table 1: Gene targets most commonly mutated in CHIP**

| **Target** | **Function** | **Mutation type(s)** |
|---|---|---|
| **TET2** | Methylcytosine dioxygenase catalyzing conversion of 5mC to 5hmC | **• Loss of function** |
| **DNMT3A** | DNA methyltransferase predominantly performing *de novo* genome-wide methylation. | • **Loss of function** |
| | | • **Dominant negative** |
| | | • **Undetermined** |
| **ASXL1** | Chromatin-binding protein regulating transcription through nuclear hormone receptors, such as retinoic acid receptors and peroxisome proliferator-activated receptor γ. Regulates PR-DUB which mediates post-translational histone modifications | • **Gain of function** (via truncated protein) |
| | | • **Dominant negative** |
| | | • **Undetermined** (multiple nonsense mutations and frameshifts) |
| **JAK2** | Signaling kinase interacting with a variety of receptors - prolactin receptor, thrombopoietic receptor, and interferon-γ involved in cell growth, development, differentiation, and histone modification. | • **Gain of function** (constitutive engagement via V617F) |
| **SF3B1** | Component of U2 snRNP which binds to pre-mRNA and is involved in RNA splicing regulation. | • **Undetermined** (multiple mutation hotspots) |
| **SRSF2** | Interacts with pre-mRNA and spliceosomal components to promote RNA splicing. | • **Gain of function** |
| **TP53** | Tumor suppressor - Transcription factor regulating cell cycle arrest, apoptosis, senescence, DNA repair, and metabolism changes in response to diverse cellular stresses. | • **Loss of function** |
| | | • **Undetermined** |
| **PPM1D** | A phosphatase, PP2C family member induced by p53, functions to deactivate and degrade p53, thus forming a negative feedback loop attenuating p53 activation. | • **Gain of function** (via truncated protein) |
| **RUNX1** | A transcription factor regulating the differentiation of hematopoietic stem cells | • **Loss of function** |
| | | • **Dominant negative** (via R174Q) |
| | | • **Undetermined** (via translocation and chimeric transcripts) |

| | | |
|---|---|---|
| The listed genetic targets have been associated with CHIP. Due to the field's novelty, there is limited functional data directly linking specific genetic mutations with CHIP. | | |

Wherever alternatives for single separable features such as, for example, a compound or a medical indication are laid out herein as "embodiments", it is to be understood that such alternatives may be combined freely to form discrete embodiments of the invention disclosed herein. Thus, any of the alternative embodiments for a compound may be combined with any of the alternative embodiments of a medical indication mentioned herein.

The invention further encompasses the following items.

### Items

1. A compound selected from lanatoside C, clopidogrel, and R406 for use in treatment of a disease associated with Cardiovascular Disease-Clonal Hematopoiesis of Indeterminate Potential (CVD-CHIP), particularly wherein the disease is characterized by the presence of myeloid cells comprising a mutation in one or several genes selected from TET2 (encoding Tet methylcytosine dioxygenase 2), DNMT3A (encoding DNA (cytosine-5)-methyltransferase 3A), ASXL1 (encoding ASXL Transcriptional Regulator 1), JAK2 (encoding Janus kinase 2), SF3B1 (encoding Splicing Factor 3b Subunit 1), SRSF2 (encoding Serine And Arginine Rich Splicing Factor 2), TP53 (encoding p53), PPM1D (encoding Protein Phosphatase, Mg2+/Mn2+ Dependent 1D), and RUNX1 (encoding Runt-related transcription factor 1), more particularly a mutation in TET2.
2. A compound selected from lanatoside C, clopidogrel, and R406 for use in prevention of a myeloid cell leukemia, particularly acute myeloid leukemia (AML), wherein the disease is characterized by the presence of myeloid cells comprising a mutation in one or several genes selected from TET2 (encoding Tet methylcytosine dioxygenase 2), DNMT3A (encoding DNA (cytosine-5)-methyltransferase 3A), ASXL1 (encoding ASXL Transcriptional Regulator 1), JAK2 (encoding Janus kinase 2), SF3B1 (encoding Splicing Factor 3b Subunit 1), SRSF2 (encoding Serine And Arginine Rich Splicing Factor 2), TP53 (encoding p53), PPM1D (encoding Protein Phosphatase, Mg2+/Mn2+ Dependent 1D), and RUNX1 (encoding Runt-related transcription factor 1), more particularly a mutation in TET2.
3. A compound selected from lanatoside C, clopidogrel, and R406 for use in prevention of heart disease,
   particularly for use in prevention of a condition selected from heart valve disease, cardiac inflammation, heart failure, and pathologic cardiac hypertrophy,
   wherein the disease is characterized by the presence of myeloid cells comprising a mutation in one or several genes selected from TET2 (encoding Tet methylcytosine dioxygenase 2), DNMT3A (encoding DNA (cytosine-5)-methyltransferase 3A), ASXL1 (encoding ASXL Transcriptional Regulator 1), JAK2 (encoding Janus kinase 2), SF3B1 (encoding Splicing Factor 3b Subunit 1), SRSF2 (encoding Serine And Arginine Rich Splicing Factor 2), TP53 (encoding p53), PPM1D (encoding Protein Phosphatase, Mg2+/Mn2+ Dependent 1D), and RUNX1 (encoding Runt-related transcription factor 1), more particularly a mutation in TET2.
4. A compound selected from lanatoside C, clopidogrel, and R406 for use in prevention of a condition selected from atherosclerosis, hypertension, diabetes, hyperlipidemia, and a high LDH level (>200 U/L), wherein the disease is characterized by the presence of myeloid cells comprising a mutation in one or several genes selected from TET2 (encoding Tet methylcytosine dioxygenase 2), DNMT3A (encoding DNA (cytosine-5)-methyltransferase 3A), ASXL1 (encoding ASXL Transcriptional Regulator 1), JAK2 (encoding Janus kinase 2), SF3B1 (encoding Splicing Factor 3b Subunit 1), SRSF2 (encoding Serine And Arginine Rich Splicing Factor 2), TP53 (encoding p53), PPM1D (encoding Protein Phosphatase, Mg2+/Mn2+ Dependent 1D), and RUNX1 (encoding Runt-related transcription factor 1), more particularly a mutation in TET2.
5. A compound selected from lanatoside C, clopidogrel, and R406 for use in prevention of a condition selected from hepatic disease, renal disease, peptic ulcer disease, hypoalbuminemia (albumin level <3.5 g/dL), and thrombocytopenia (platelet count of <20 x 103 cells/µL), wherein the disease is characterized by the presence of myeloid cells comprising a mutation in one or several genes selected from TET2 (encoding Tet methylcytosine dioxygenase 2), DNMT3A (encoding DNA (cytosine-5)-methyltransferase 3A), ASXL1 (encoding ASXL Transcriptional Regulator 1), JAK2 (encoding Janus kinase 2), SF3B1 (encoding Splicing Factor 3b Subunit 1), SRSF2 (encoding Serine And Arginine Rich Splicing Factor 2), TP53 (encoding p53), PPM1D (encoding Protein Phosphatase, Mg2+/Mn2+ Dependent 1D), and RUNX1 (encoding Runt-related transcription factor 1), more particularly a mutation in TET2.
6. The compound for use according to any one of the preceding items, wherein the mutation in TET2 is a mutation which causes expression of a loss-of-function TET2 protein or a TET2 protein with reduced activity, or a mutation which lowers or abrogates expression of TET2, particularly the mutation introduces a stop codon into the TET2 gene leading to expression of a loss-of-function TET2 protein.
7. The compound for use according to any one of the preceding items, wherein the myeloid cells comprise functional expression of the genes FLT3, N/KRAS, KIT, PTPN11, TP53, PHF6, SRSF2, STAG2, EZH2, and RAD21.
8. The compound for use according to any one of the preceding items, wherein the myeloid cells comprise functional expression of the genes PML-RARA, MYH11-CBFB, RUNX1-RUNX1T1, MLLT3-KMT2A, DEK-NUP214, RUNX1, NPM1, CEBPA, GATA2.
9. The compound for use according to any one of the preceding items, wherein the compound is lanatoside C.
10. The compound for use according to any one of the preceding items 1 to 8, wherein the compound is clopidogrel.
11. The compound for use according to any one of the preceding items 1 to 8, wherein the compound is R406.

The invention is further illustrated by the following examples and figures, from which further embodiments and advantages can be drawn. These examples are meant to illustrate the invention but not to limit its scope.

*Description of the Figures*

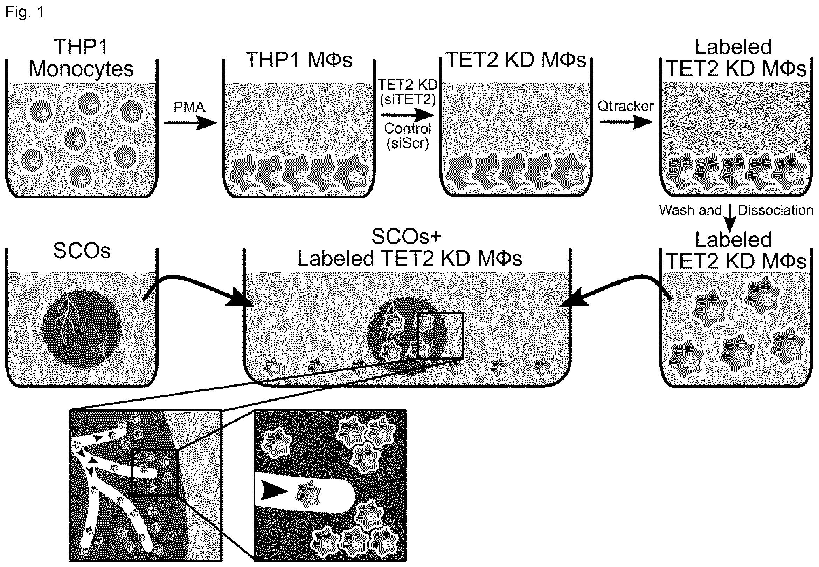

- **Figure 1**: shows the scheme for the disease model used for the invention.

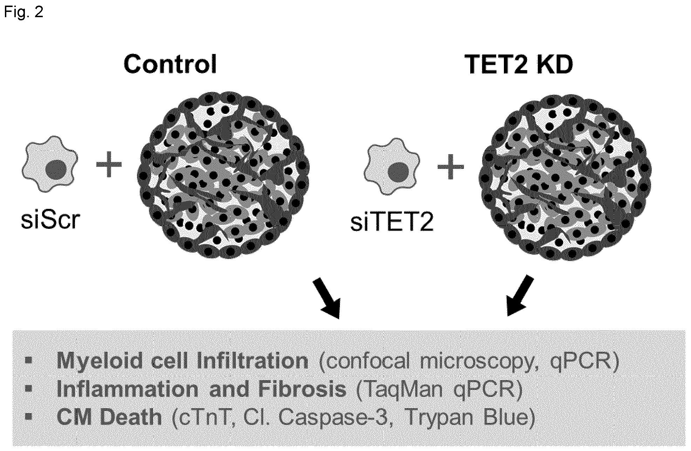

- **Figure 2**: shows the comparative concept behind the screening TET2 CHIP model and the parameters evaluated.

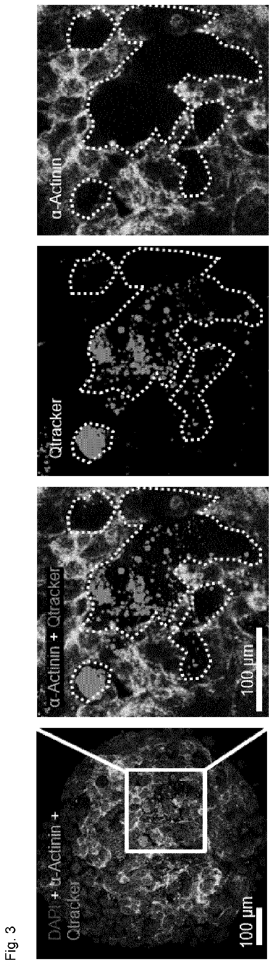

- **Figure 3**: shows effective cell labelling and myeloid cell infiltration. Tracked/Stained myeloid cells infiltrate a cardiac organoid. The Qtracker staining and subsequent tracking of myeloid cells enables monitoring and analysis of their infiltration into cardiac organoids.

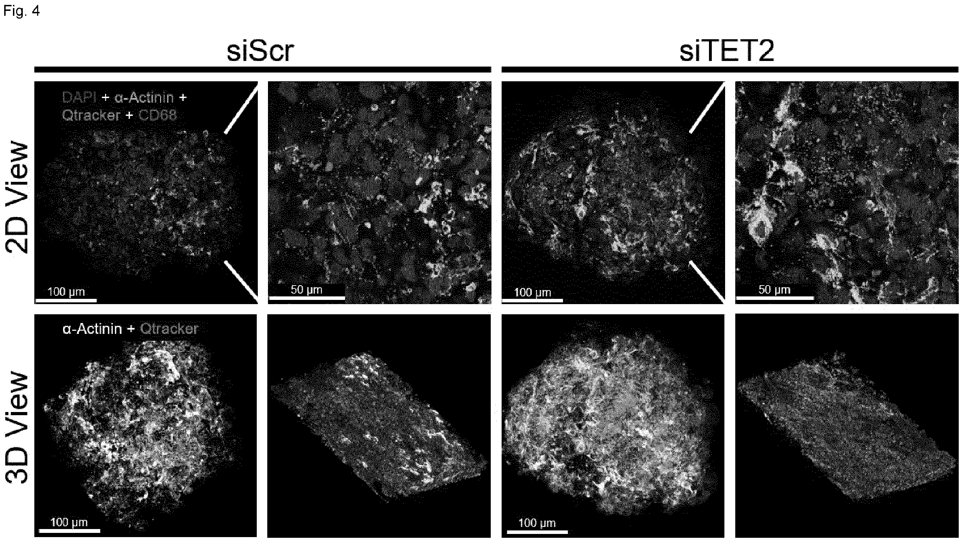

- **Figure 4**: shows higher infiltration of TET2-deficient myeloid cells into cardiac tissue under cardiopathology conditions (siTET2). Monitoring of myeloid cell infiltration into organoids shows increased infiltration of siTET2 myeloid cells as compared to Control (siScr), replicating the increased myeloid cell infiltration observed in CHIP patients.

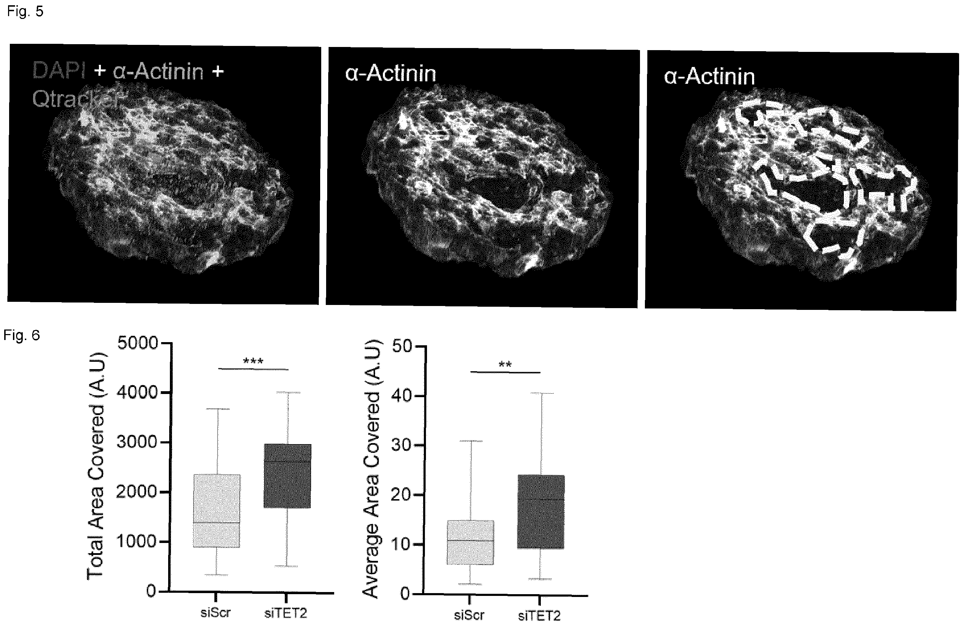

- **Figure 5**: shows evident myeloid cell infiltration in organoids. Infiltration areas are devoid of cardiomyocytes. Notable myeloid cell clustering is visible. 3D rendering of organoids with labeled infiltrating myeloid cells. The absence of cardiomyocytes from infiltration areas further confirms effective myeloid cell staining and tracking. Additionally, myeloid cell clustering is demonstrated.
- **Figure 6**: shows quantification of myeloid cell infiltration in organoids. Increased area covered with myeloid cells in siTET2 organoids. Quantification of myeloid cell infiltration in organoids shows increased organoid area covered with myeloid cells in organoids cocultured with siTET2 myeloid cells compared to Control (siScr) (** p <0.01, *** p<0.01).

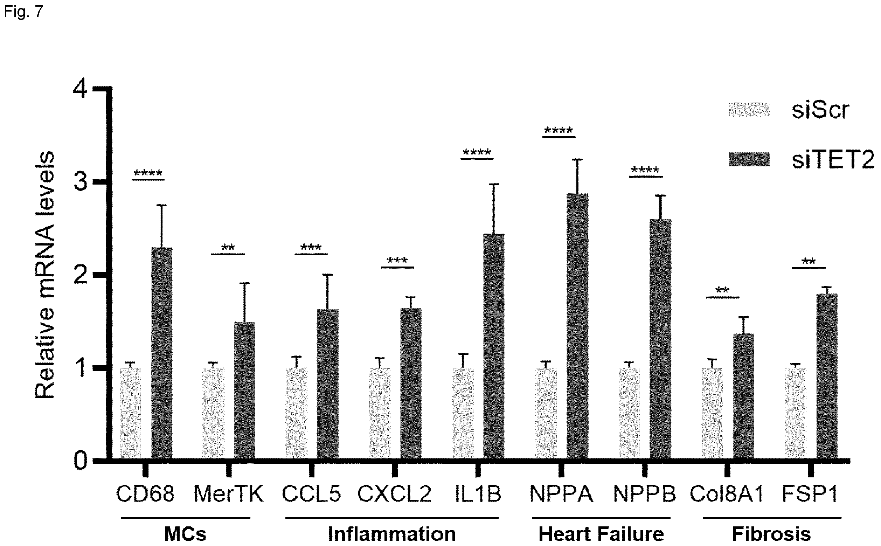

- **Figure 7**: shows increased levels of myeloid, inflammation, heart failure and fibrosis markers in cardiac organoids infiltrated with CHIP (siTET2) compared to healthy (siScr) organoids (* p <0.05, ** p <0.01, *** p <0.001, **** p <0.0001).

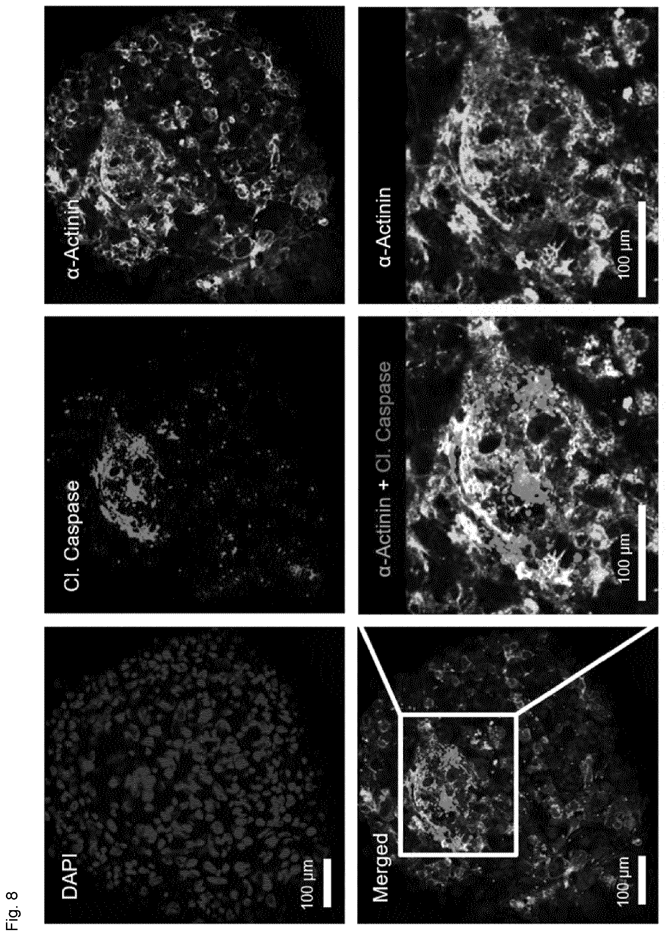

- **Figure 8**: shows cardiomyocyte apoptosis. Evident colocalization of cardiomyocytes (CMs) and Cleaved-Caspase 3 apoptosis staining demonstrates that it is the CMs that undergo apoptosis.

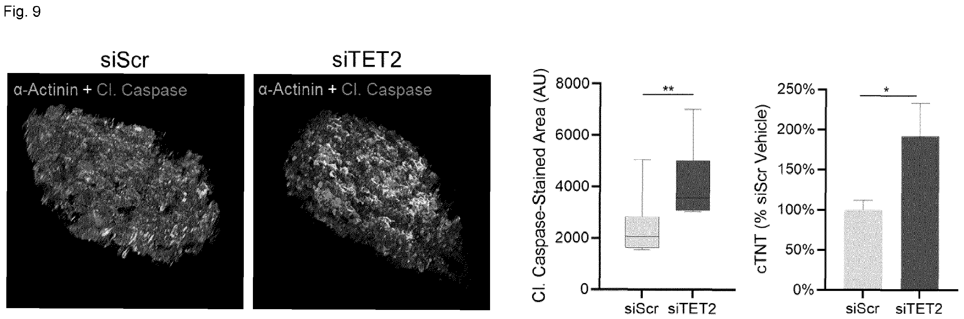

- **Figure 9**: shows s 3D rendering where organoids infiltrated by siTET2 myeloid cells experience higher levels of cardiomyocyte apoptosis than those infiltrated with healthy (siScr) myeloid cells (left). Quantification of cleaved caspase 3 staining shows increased apoptosis in the siTET3 KD group as compared to control (siScr, middle). The increased cardiomyocyte death is further confirmed by an increase in the prognostic cardiac biomarker cTNT in the siTET2 group (right). (* p <0.05, ** p <0.01).

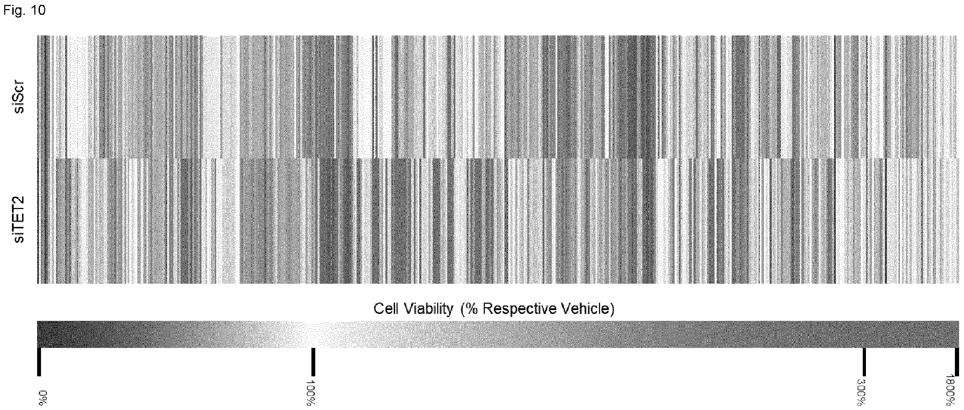

- **Figure 10**: shows cell viability of CHIP (siTET2) and healthy (siScr) myeloid cells treated with a library of CVD drugs. The screen led to the identification of compounds that decrease the viability of CHIP cells without compromising the viability of healthy cells.

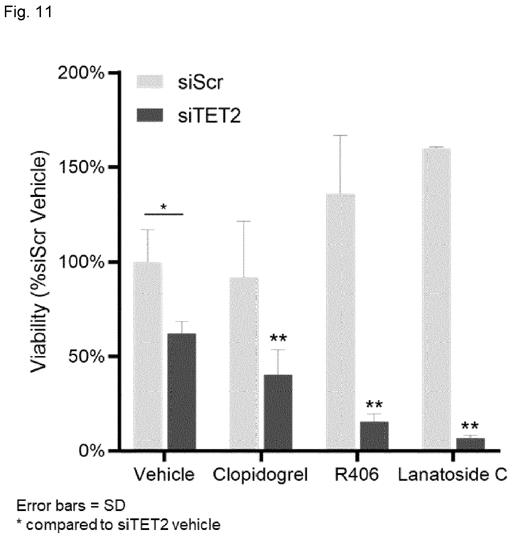

- **Figure 11**: shows significantly decreased viability for myeloid cells treated with Clopidogrel, R406 or Lanatoside C. A decrease in viability of myeloid cells upon treatment is observed in the CHIP (siTET2) condition, without a decrease in viability for healthy (siScr) cells. (* p <0.05, ** p <0.01)

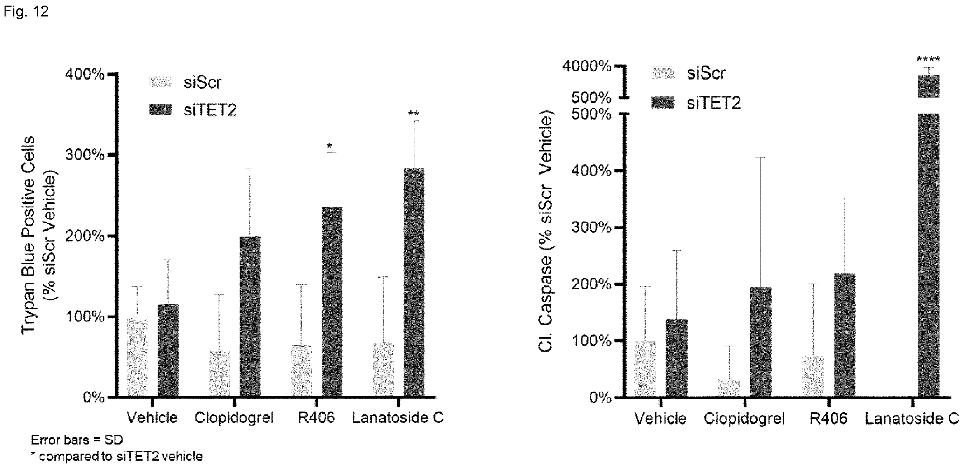

- **Figure 12**: shows selective targeting of CHIP myeloid cells upon treatment with Clopidogrel, R406 or Lanatoside C. An increase in apoptosis of myeloid cells upon treatment is observed in the CHIP (siTET2) condition, without an increase in apoptosis for healthy (siScr) cells, measured by Trypan Blue (left) and Cl. Caspase (right). (* p <0.05, ** p <0.01, *** p <0.001, **** p <0.0001).

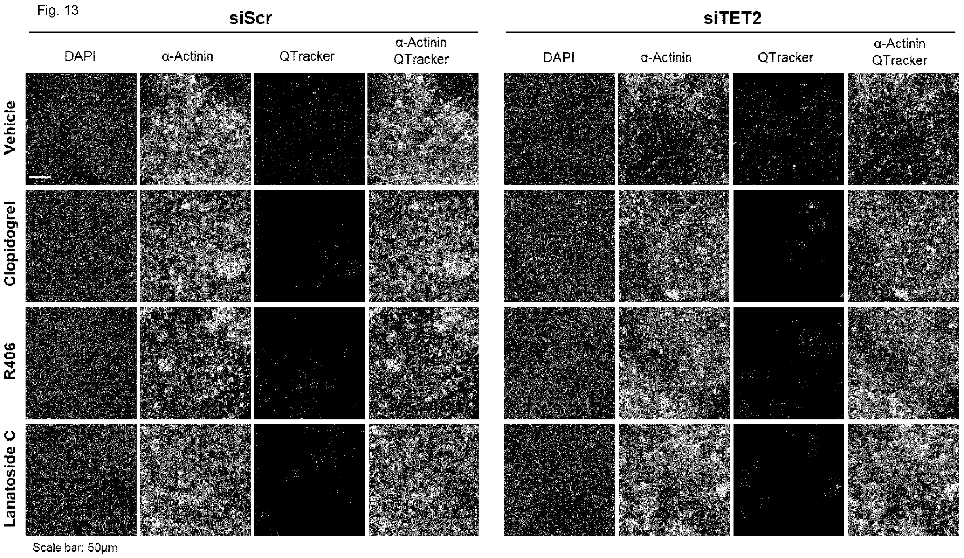

- **Figure 13**: shows a recovery of myeloid infiltration levels in CHIP-infiltrated cardiac organoids (siTET2) back to healthy (siScr) levels upon treatment with Clopidogrel, R406 or Lanatoside C. The decreased levels of Qtracker staining in the treated conditions indicate there is a lower number of myeloid cells in the cardiac organoids.

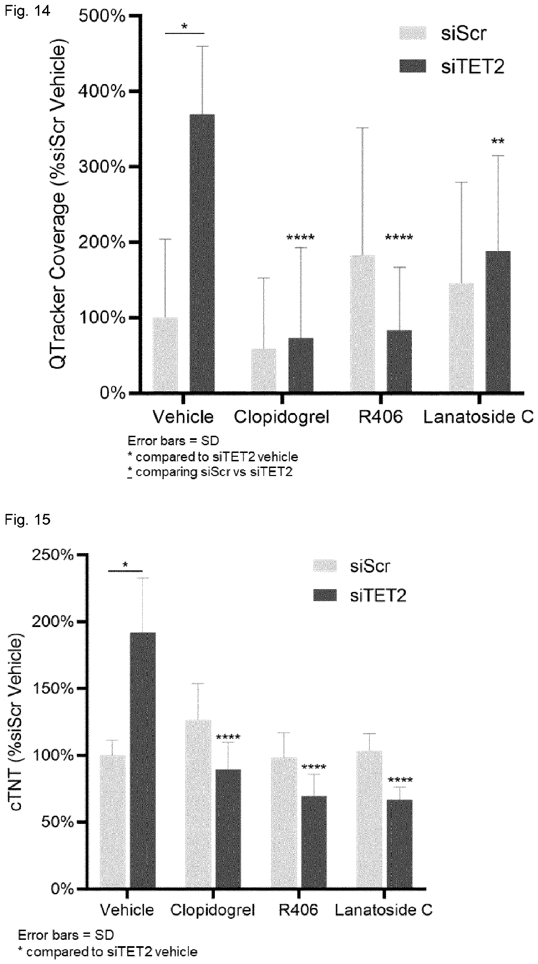

- **Figure 14**: shows a recovery of myeloid infiltration levels in CHIP-infiltrated cardiac organoids (siTET2) upon treatment with Clopidogrel, R406 or Lanatoside C back towards healthy (siScr) levels. The decreased levels of Qtracker Coverage indicate there is a lower number of myeloid cells in the cardiac organoids. (* p <0.05, ** p <0.01, *** p <0.001, **** p <0.0001).
- **Figure 15**: shows a recovery of the levels of prognostic cardiac biomarker cTNT upon treatment of CHIP-infiltrated cardiac organoids (siTET2) with Clopidogrel, R406 or Lanatoside C (compared to vehicle control) back to healthy (siScr) levels. (* p <0.05, ** p <0.01, *** p <0.001, **** p <0.0001).

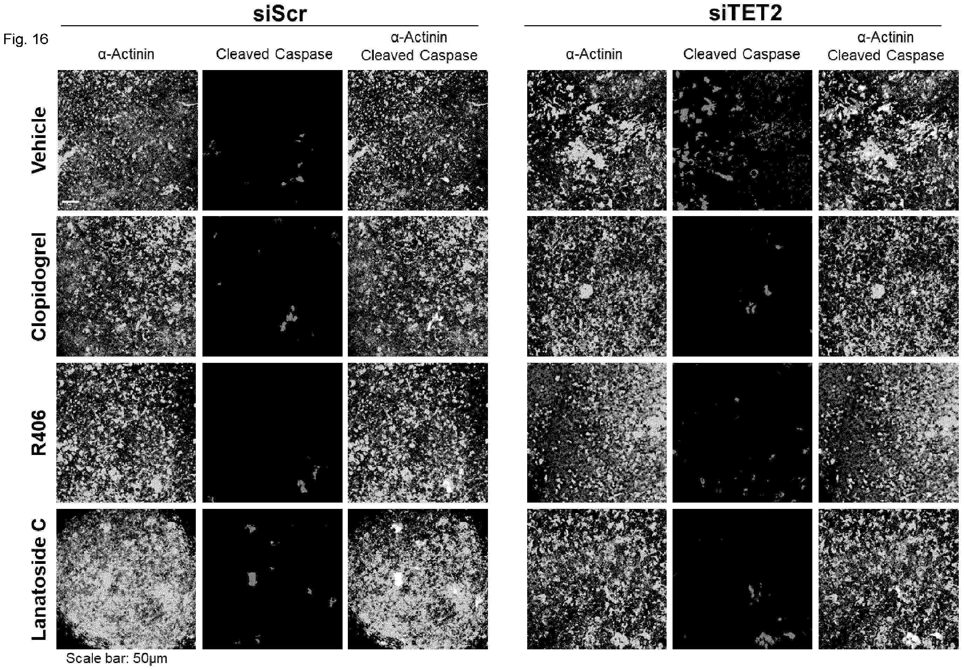

- **Figure 16**: shows a recovery of cardiomyocyte apoptosis levels in CHIP-infiltrated cardiac organoids (siTET2) upon treatment with Clopidogrel, R406 or Lanatoside C (compared to vehicle control) back to healthy (siScr) levels. The lower level of Cleaved Caspase staining colocalized with α-Actinin indicates a decrease in cardiomyocyte apoptosis in the cardiac organoid upon drug treatment.

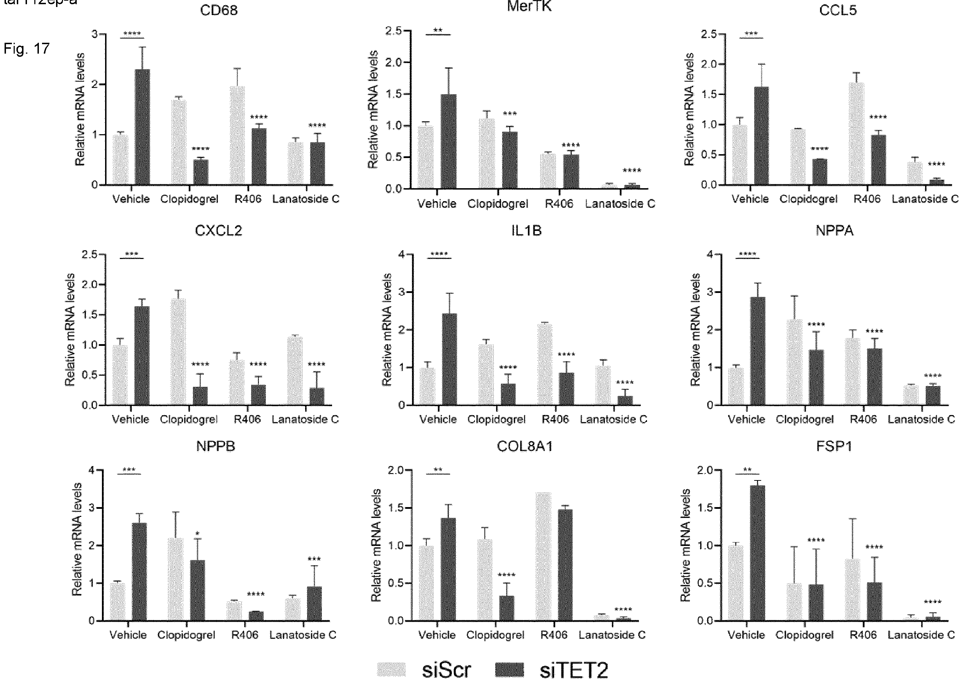

- **Figure 17**: shows a predominant decrease of inflammation, myeloid, and fibrosis markers in CHIP-infiltrated cardiac organoids upon treatment with Clopidogrel, R406 or Lanatoside C compared with vehicle control. Data is normalized to healthy vehicle control (1.0). (* p <0.05, ** p <0.01, *** p <0.001, **** p <0.0001).

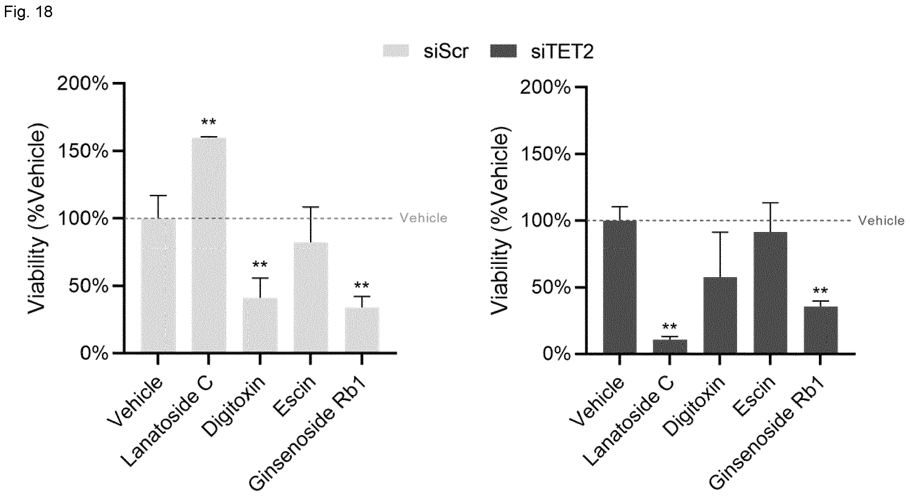

- **Figure 18**: shows compounds with similar chemistry (Digitoxin, Escin, Ginsenoside Rb1) and/or target (Digitoxin) as Lanatoside C screened for their effect on viability on healthy (siScr, left) or CHIP (siTET2, right) cells. Of the similar drugs that managed to decrease viability of CHIP cells below Vehicle levels, Ginsenoside Rb1 was found to do so significantly (Mann-Whitney statistical tests, *p<0.05, **p<0.01). However, it also decreases the viability of healthy cells making it unsuitable for the purpose in question.

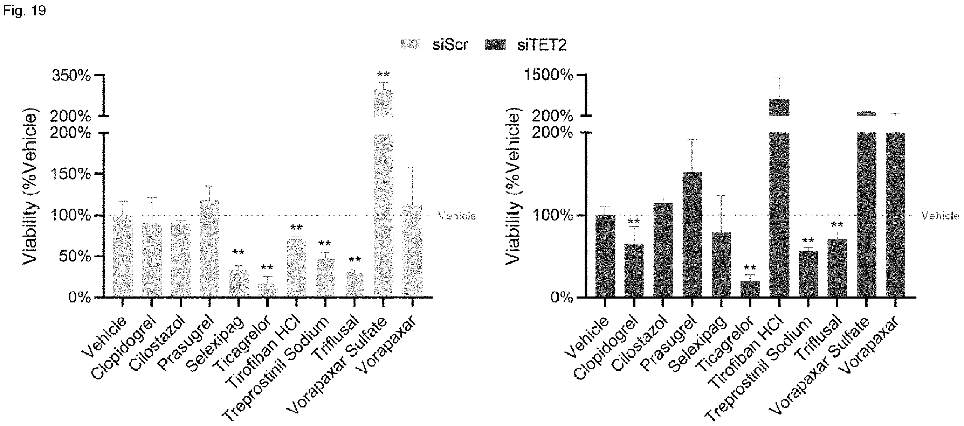

- **Figure 19**: shows compounds with similar ATC classification (Cilostazol, Prasugrel, Selexipag, Ticagrelor, Tirofiban HCI, Treprostinil Sodium, Trifusal, Vorapax Sulfate and Vorapax) and/or target (Prasugrel, Ticagrelor) as Clopidogrel screened for their effect on viability on healthy (siScr, left) or CHIP (siTET2, right) cells. Of the similar drugs that managed to decrease viability of CHIP cells below Vehicle levels, Ticagrelor, Treprostinil Sodium and Triflusal were found to do so significantly (Mann-Whitney statistical tests, *p<0.05, **p<0.01). However, they also decrease the viability of healthy cells making them unsuitable for the purpose in question.

### Examples

### Example 1: Model Validation

The inventors have recapitulated a TET2-based CHIP model with this system. TET2 is one of the most frequently mutated genes in CHIP patients. The mutations occur mostly on a single genetic allele, and despite the variety in mutation types detected in CHIP patients, they all lead to a loss of function of the gene product.

The inventors have used siRNA to knock down TET2 (siTET2) in THP1 myeloid cells (MCs). **Figure 1** shows the method for generation of the disease model. **Figure 2** summarizes the model design and the methods of model validation, as well as for drug screening and validation.

These MCs were utilized in the coculturing system with self-organized human cardiac organoids, where they were tracked using a Qtracker stain. **Figure 3** shows images of MCs in the cardiac organoid, demonstrating successful selective tracking of the infiltrating cells, enabling monitoring and analysis of the degree of infiltration, their location in the organoid and other parameters.

When control (siScr) and CHIP (siTET2) MCs were allowed to infiltrate cardiac organoids in cardiopathologic conditions, a higher infiltration caused by CHIP (siTET2) MCs was observed than of healthy (siScr) MCs, as the representative images in **Figure 4** show. This replicates the increased MCs infiltration observed in CHIP patients. **Figure 5** shows a 3D rendering of organoids with labeled infiltrating MCs. The absence of CMs from infiltration areas further confirms effective MCs straining and tracking. Additionally, notable MCs clustering is visible.

The inventors quantified the levels of MCs infiltration in organoids. As shown in **Figure 6****,** the area covered by MCs is increased when siTET2 MCs infiltrate an organoid compared to control (siScr) cells (30+ regions of interest from multiple different organoids assessed, *** P<0.001, ** p<0.01).

**Figure 7** shows increased relative mRNA levels of myeloid markers CD68 and MerTK, and inflammation markers CXCL2, CCL5, and IL1B, in CHIP-infiltrated organoids (siTET2) compared to those infiltrated with healthy myeloid cells (siScr) (* p <0.05, ** p <0.01, *** p <0.001, **** p <0.0001). This demonstrates increased infiltration and inflammation in the siTET2 model, matching the phenotype observed in CHIP patients. In addition, relative mRNA levels of heart failure markers NPPA and NPPB, and Fibrosis markers Col8A1 and FSP1 are increased in siTET2 compared to control (siScr), demonstrating increased cardiac damage and initiation of fibrotic processes. This indicates increased fibrosis, which is an indicator of tissue damage and the body's attempt to make up for the cell loss by fibroblast expansion, which ends up compromising the function of the heart.

To assess the CM death, the inventors used cleaved caspase staining which labels apoptotic cells. The colocalization of this staining and the α-Actinin staining (for CMs) shown in **Figure** 8 indicates that it is indeed CMs that undergo apoptosis. **Figure 9** shows a 3D rendering where it is evident that organoids infiltrated by siTET2 MCs experience higher levels of CM apoptosis compared to those infiltrated by control cells. **Figure 9** also shows the quantification of area covered by cleaved caspase 3, and an evident increase of CM apoptosis in organoids infiltrated with siTET2 MCs (** p <0.01). The increase in CM death in the TET2 KD group is further verified by an increase in cardiac troponin T (cTNT), a prognostic cardiac damage clinical biomarker (* p <0.05). These indicators together demonstrate that this model recapitulates the cardiomyocyte loss observed in CHIP patients.

In summary, increased infiltration, inflammation and cardiomyocyte death have been accurately recapitulated by this disease model utilizing the invention.

### Example 2: Drug screening and validation

The established disease model can subsequently be used for drug screening. Thus, a synthetic lethality screening of a CVD (cardiovascular disease) drug library was performed, treating CHIP (siTET2) or healthy (siScr) myeloid cells.

Figure 10 shows a heatmap of the viability changes caused by the drugs. Figure 11 shows the effect on viability of a total of 3 hits (Clopidogrel, R406 and Lanatoside C) which were found to selectively inhibit the viability of CHIP myeloid cells (siTET2) without compromising the viability of healthy (siScr) cells.

Figure 12 shows the evaluation of treatment of healthy (siScr) and CHIP (siTET2) myeloid cells with the 3 hit drugs, demonstrating that Clopidogrel, R406 and Lanatoside C increase the levels of cell death of CHIP myeloid cells (Trypan Blue Assay) and apoptosis in particular (CI. Caspase Assay), without inflicting harm on the healthy (siScr) cells. This shows that the drugs in question are able to selectively target and kill CHIP cells.

Next, the inventors have seen before that the infiltration of myeloid cells into organoids, which is higher for siTET2 cells compared to siScr cells without drug treatment (Vehicle). This effect was rescued, by treatment of CHIP-infiltrated organoids (siTET2) with each of the 3 hit drugs (Clopidogrel, R406, Lanatoside C), as shown by the decreased levels of Qtracker-stained area upon treatment of siTET2 cells back to the baseline healthy levels (siScr Vehicle). Figure 14 shows the quantification of this effect (* p <0.05, ** p <0.01, *** p <0.001, **** p <0.0001).

Figure 15 shows that the levels of the prognostic marker cTNT which increase upon CHIP-cell infiltration into cardiac organoids (siTET2) compared to the healthy condition (siScr) without treatment (Vehicle) are able to be rescued, i.e., decreased back to healthy levels upon treatment with Clopidogrel, R406 or Lanatoside C (* p <0.05, **** p <0.0001).

Figure 16 shows that the cardiomyocyte apoptosis levels which are observed to be far higher for cardiac organoids infiltrated with CHIP (siTET2) compared healthy (siScr) myeloid cells without treatment (Vehicle) are also rescued upon treatment with Clopidogrel, R406 or Lanatoside C, as evidenced by the decreased areas of Cleaved Caspase staining back towards healthy levels (siScr) upon treatment of siTET2 with the aforementioned drugs.

Figure 17 shows the gene expression observed in cardiac organoids infiltrated with CHIP myeloid cells without treatment (Vehicle) or after treatment with Clopidogrel, R406 or Lanatoside C (compared to the healthy state = 1.0). The inventors observe that the relative increases in RNA levels of myeloid, inflammatory, heart failure and fibrotic markers without treatment (Vehicle) are rescued upon treatment with the 3 aforementioned drugs back down towards healthy levels (≤1.0).

In order to investigate the specificity of the hit drugs, the inventors sought out related compounds that share properties with them which were also part of the library screening.

Figure 18 shows compounds with similar chemistry (Digitoxin, Escin, Ginsenoside Rb1) and/or target (Digitoxin) as Lanatoside C, which were screened for their effect on viability on healthy (siScr, left) or CHIP (siTET2, right) cells. Of the similar drugs that managed to decrease viability of CHIP cells below Vehicle levels, only Ginsenoside Rb1 was found to do so significantly (Mann-Whitney statistical tests, *p<0.05, **p<0.01). However, Ginsenoside Rb1 also decreases the viability of healthy cells making it unsuitable for the selective targeting of CHIP cells.

Figure 19 shows compounds with similar ATC classification (Cilostazol, Prasugrel, Selexipag, Ticagrelor, Tirofiban HCI, Treprostinil Sodium, Trifusal, Vorapax Sulfate and Vorapax) and/or target (Prasugrel, Ticagrelor) as Clopidogrel, which were screened for their effect on viability on healthy (siScr, left) or CHIP (siTET2, right) cells. Of the similar drugs that managed to decrease viability of CHIP cells below Vehicle levels, Ticagrelor, Treprostinil Sodium and Triflusal were found to do so significantly (Mann-Whitney statistical tests, *p<0.05, **p<0.01). However, they also decrease the viability of healthy cells making them unsuitable for the purpose in question.

In conclusion, the 3 identified drugs: Clopidogrel, R406 and Lanatoside C can specifically and selectively target CHIP (siTET2) myeloid cells, and consequently decrease the infiltration into cardiac tissue, as well as the levels of inflammation, fibrosis, and cardiomyocyte death within.

### Materials and Methods

### Organoid culturing

Human induced pluripotent stem cells (hiPSCs) were used for TrueCardium generation. In brief, 500 hiPSCs were cultured on ultra-low-attachment surface in medium TeSR^{™}-E8^{™} at 37°C and 5% CO2 at humidified atmosphere to form embryoid body (EB). After 2 days, EBs were differentiated to cardiac organoids (COs) by replacing medium every 48 hours with medium 1, medium 2, medium 3 (2 times) and medium 4. COs were maintained in medium 4 for 10 days by refreshing medium every second day. Medium 4 was then replaced by medium 5 for 4 days with refreshing medium every 48 h. After that COs were transferred to rotating incubator with medium 6 for 6 days with refreshing medium every second day. COs were then ready to be harvested.

### Media info:

- **Basal medium:**
   - RPMI 1640 with Glutamax
   - 1% of 100X sodium pyruvate
   - 1% of 100X penicillin/streptomycin
   - 3% B27 supplement
- **Medium 1:**
   - Basal medium
   - 50 ng/mL of Activin A
   - 2 ng/mL of BMP-4
   - 5 ng/mL of hFGF
   - 1 µM CHIR
   - 100 µM ACS-2-P
- **Medium 2:**
   - Basal medium
   - 5 ng/mL of Activin A
   - 10 ng/mL of BMP-4
   - 5 ng/mL of bFGF
   - 1 µM CHIR
   - 100 µM ACS-2-P
- **Medium 3:**
   - Basal medium
   - 5 µM IWP4
   - 200 µM ACS-2-P
- **Medium 4:**
   - Basal medium
   - 200 µM ACS-2-P

### Cell density of iPS cells in cardiac organoid:

- Optimize from 500 to 3000 iPSCs per EB
- Density of 500 iPSCs per EB was chosen as it gave best result in organoid formation.

### Timing for the cardiac organoid generation:

- Day -2: EB formation from iPSCs in microwell
- Day 0: Add Medium 1 supplemented with **Matrigel PSC grade (dilution 1:100)**
- After **exact 48h** (day 2): Add Medium 2
- After **exact 48h** (day 4): Add Medium 3
- After **exact 48h** (day 6): Change fresh Medium 3
- After **exact 48h** (day 8): Add Medium 4
- **From day 8 to day 18:** Change fresh Medium 4 every second day
- **Day 18:** Add Medium 4 supplemented **with 50nM hVEGF and 25nM hFGF**
- After **exact 48h** (day 20): Change fresh Medium 4 supplemented **with 50nM hVEGF and 25nM hFGF**
- After **exact 48h** (day 22): transfer Cardiac organoids to rotating incubator and add **Mix Medium (Medium 4: EGM2 (without FBS) at ratio 4:1)**
- From day 22 to day 28: maintain cardiac organoids in rotating incubator. Change mix medium every second day
- Day 29: Harvest cardiac organoid

### Macrophage culturing

THP-1 cells were purchased from DSMZ (ACC 16) and maintained in RPMI-1640 (Gibco) +10% Fetal Calf Serum, 1% Penicillin/Streptomycin, 1% Glutamine, 0.05 mM 2-Mercaptoethanol.

THP-1 cells were differentiated from monocytes to macrophages by supplementing the media 100 ng/mL PMA and culturing for 48 h, and then in PMA-free media for another 24 h. For routine culturing, cells were maintained at 37°C, 5% CO₂, 20% O₂. In cardiopathologic culturing, both THP1 cells and organoids were maintained hypoxic conditions at 37°C, 5% CO₂, 1% O₂.

### TET2 Knockdown

Transfection of THP-1 cells with siRNA purchased as ON-TARGETplus pools from Horizon Discovery (TET2 siRNA pool containing: ACAAGAAAGUAGAGGGUAU (SEQ ID NO 001), ACACCUAGUUUCAGAGAAU (SEQ ID NO 002), CCUCAGAAUAAUUGUGUGA (SEQ ID NO 003), CAGCAAAGGUACUUGAUAC (SEQ ID NO 004); Control siRNA pool containing: UGGUUUACAUGUUGUGUGA (SEQ ID NO 005), UGGUUUACAUGUUUUCCUA (SEQ ID NO 006)).

Working solution of 50µM siRNA was prepared in siRNA Buffer (ThermoFisher). 2 µL of 50µM siRNA were mixed with 500 µL OptiMEM and 6.25µL LTX Reagent PLUS (ThermoFisher) and incubated at room temperature (RT) for 15 min. 6.25 µL Lipofectamine LTX was added, mixed, and incubated for 30 min at RT. 500 µL were then added to the target culture and the culture was incubated at 37°C, 5% CO₂. Passaging and/or media change was performed after 24 h.

### Organoid and Macrophage Coculture

Macrophages were differentiated from THP1 monocytes, then treated with siRNAs (18 h incubation), washed, and labeled with Qtracker^{™} 655 Cell Labeling Kit (ThermoFisher) for 1 h according to the manufacturer's instructions, washed with PBS, and dissociated with 0.05% trypsin. Macrophages were then added to organoids and cultured at 37°C, 5% CO₂, and 1%O₂. Cultures were maintained in 96-well U-bottom plates.

Immunostaining, RNA extraction and cTNT assays were run after 3 days of culturing.

### Immunofluorescent staining

Immunofluorescent stainings were performed as described before (Krishnan et al., Cell Metab 9, 512-524 (2009)). After an overnight PFA fixation, the organoids were washed with PBS, then treated with 1% TritonX-100 (in PBS) for 45 minutes. Afterwards, they are incubated with PBS for 10 minutes for washing. 5% horse serum (in PBS) is added to block and incubated for 45 minutes (RT) Primary antibody in 2% horse serum + 0.0002% TritonX-100 was added and the samples were incubated at 4°C overnight. Primary antibodies: Anti-Sarcomeric-actinin (Ab68167, anti-human), Anti-Cleaved Caspase-3 (Ab32042, anti-human). Samples were washed with PBS + 0.002% TritonX-100 6x10min. Secondary antibodies and additional stains were added and incubated for 4h at RT: AlexaFlour 488 (Invitrogen, A11070), AlexaFlour 555 (Invitrogen, A21425), DAPI, Qtracker labelling 655 (Invitrogen, Q25021 MP). Samples were washed by incubating with PBS+0.002%TritonX-100 for 2-3 hours at RT), and mounted on Superfrost Plus Adhesion Slides (ThermoFisher) sing transparent polish to seal the slide sides to the coverslips (Roth). Samples were imaged using SP8 Lightning Leica Confocal Microscope, using a 40x objective (for a total of 800x and 300x magnification with zoom factors and eye lenses). Apoptosis quantification was performed by analyzing the Cleaved-Caspase-3-stained area using FIJI (ImageJ).

### cTNT Assay

The cTNT assay was performed using the Human Cardiac Troponin T ELISA^{®} Kit (ab223860, Abcam) following the manufacturer's instructions.

### Synthetic Lethality Screen

THP-1 monocytes and macrophages were cocultured overnight (18 h) with siRNA. They were then washed with PBS and used for either TET2 knockdown quality control (as described below) or for the drug screening. For the screening, cells were transferred from tissue culture flasks to 384-well plates. Drugs were added to a final concentration of 100 µM and incubated for 48 hours. An alamarBlue Cell Viability assay (Invitrogen) with a 4 h incubation was performed according to the manufacturer's instructions.

### TET2 KD Quality Control (qPCR)

Quality control was performed by extracting RNA from the cells using the RNeasy Mini Kit (Qiagen) following the manufacturer's instructions, and performing qPCR using the RNA to cDNA EcoDry^{™} Premix (TakaraBio) following the manufacturer's instructions with the following TET2 primers: GTGAGGCTGCAGTGATTGTG (SEQ ID NO 007), GATTGGTGAGCGTGCCGTAT (SEQ ID NO 008). The samples were measured and analyzed using Applied Biosystems^{™} PCR system (4376592) and the associated Step One (v2.3) software. Both SYBR^{™} Green Master Mix (4385612, Applied Biosystems) and TaqMan^{™}

## Claims

1. A compound selected from lanatoside C, clopidogrel, and R406 for use in treatment of a disease associated with Cardiovascular Disease-Clonal Hematopoiesis of Indeterminate Potential (CVD-CHIP), particularly wherein the disease is **characterized by** the presence of myeloid cells comprising a mutation in TET2.

2. A compound selected from lanatoside C, clopidogrel, and R406 for use in prevention of a myeloid cell leukemia, particularly acute myeloid leukemia (AML), wherein the disease is **characterized by** the presence of myeloid cells comprising a mutation in TET2.

3. A compound selected from lanatoside C, clopidogrel, and R406 for use in prevention of heart disease,
particularly for use in prevention of a condition selected from heart valve disease, cardiac inflammation, heart failure, and pathologic cardiac hypertrophy,
wherein the disease is **characterized by** the presence of myeloid cells comprising a mutation in TET2.

4. A compound selected from lanatoside C, clopidogrel, and R406 for use in prevention of a condition selected from atherosclerosis, hypertension, diabetes, hyperlipidemia, and a high LDH level (>200 U/L), wherein the disease is **characterized by** the presence of myeloid cells comprising a mutation in TET2.

5. A compound selected from lanatoside C, clopidogrel, and R406 for use in prevention of a condition selected from hepatic disease, renal disease, peptic ulcer disease, hypoalbuminemia (albumin level <3.5 g/dL), and thrombocytopenia (platelet count of <20 x 103 cells/µL), wherein the disease is **characterized by** the presence of myeloid cells comprising a mutation in TET2.

6. The compound for use according to any one of the preceding claims, wherein the mutation in TET2 is a mutation which causes expression of a loss-of-function TET2 protein or a TET2 protein with reduced activity, or a mutation which lowers or abrogates expression of TET2, particularly the mutation introduces a stop codon into the TET2 gene leading to expression of a loss-of-function TET2 protein.

7. The compound for use according to any one of the preceding claims, wherein the myeloid cells comprise functional expression of the genes FLT3, N/KRAS, KIT, PTPN11, PHF6, SRSF2, STAG2, EZH2, and RAD21.

8. The compound for use according to any one of the preceding claims, wherein the myeloid cells comprise functional expression of the genes PML-RARA, MYH11-CBFB, RUNX1-RUNX1T1, MLLT3-KMT2A, DEK-NUP214, RUNX1, NPM1, CEBPA, GATA2.

9. The compound for use according to any one of the preceding claims, wherein the compound is lanatoside C.

10. The compound for use according to any one of the preceding claims 1 to 8, wherein the compound is clopidogrel.

11. The compound for use according to any one of the preceding claims 1 to 8, wherein the compound is R406.
